# EUROPÄISCHE PATENTANMELDUNG

(11) **EP 1 774 951 A1**
(43) Veröffentlichungstag der Anmeldung: **18.04.2007**
(21) Anmeldenummer: 05109440.7
(22) Anmeldetag: 11.10.2005
(51) Int. Cl.: A61K 8/02

(54) **Flüssiges Reinigungs- und Pflegemittel**

(71) Anmelder: Dux S.A., 1019 Luxembourg (LU)
(72) Erfinder: Giebel, Alexander, 54295, Trier (DE)
(74) Vertreter: Jönsson, Hans-Peter

(57) **Zusammenfassung**

Gegenstand der Erfindung ist ein im Wesentlichen parfumfreies, alkoholfreies, flüssiges Reinigungs- und Pflegemittel auf Wasserbasis für den menschlichen Intim- und Analbereich sowie wie für Gegenstände die damit gegebenenfalls in Kontakt treten. Weitere Gegenstände der Erfindung sind ein entsprechendes Reinigungs- und Pflegetuch sowie eine entsprechende Pumpsprühflasche.

## Beschreibung

Gegenstand der Erfindung ist ein im Wesentlichen parfumfreies, alkoholfreies, flüssiges Reinigungs- und Pflegemittel auf Wasserbasis für den menschlichen Intim- und Analbereich sowie wie für Gegenstände die damit gegebenenfalls in Kontakt treten. Weitere Gegenstände der Erfindung sind ein entsprechendes Reinigungs- und Pflegetuch sowie eine entsprechende Pumpsprühflasche.

DE 600 19 505 T2 beschreibt mikrobizide Wirksubstanzen, die sich zur antimikrobiellen Ausrüstung von Kunststoffen eigenen. Auch wird beschrieben, Vliesware mit antimikrobiellen Eigenschaften auszurüsten.

DE 600 03 147 T2 betrifft eine wasserdispergierbare Faserfläche aus fibrillierten Reyonfasern mit verschiedenen Längenverteilungen. Ein ähnliches Faserflächenprodukt wird in DE 600 09 179 T2 beschrieben.

Aus DE 600 03 480 T2 ist ein wasserdispergierbares Vlies bekannt, dass Fasern verschiedener Länge aus regenerierter Zellulose enthält.

DE 197 29 921 A1 beschreibt die Verwendung eines bekannten Reinigungs- und Pflegetuchs für den Anal- und Intimbereich zur Reinigung, Pflege und Desinfektion des Stomabereichs, wobei das Reinigungs- und Pflegetuch eine mit einer Wirkstofflösung getränkte textile Schicht umfasst und wobei die Wirkstofflösung Bakterizide, reinigende und pflegende Komponenten umfasst.

Dem Gegenüber besteht die Aufgabe der vorliegenden Erfindung in der Bereitstellung eines im Wesentlichen parfumfreien, alkoholfreien, flüssigen Reinigungs- und Pflegemittels auf Wasserbasis für den menschlichen Intim- und Analbereich sowie für Gegenstände, die damit gegebenenfalls in Kontakt treten. Ein entsprechendes Reinigungs- und Pflegemittel soll zur medizinischen Infektionsprophylaxe dienen, intensiv antimirkobiell gegen Viren beispielsweise Hepatitis B oder HIV sowie Bakterien und Pilze wirken. Dementsprechend ist weiterhin eine Randbedingung die medizinische Reinheit, die Freiheit von Alkohol und die Schleimhautverträglichkeit. Darüber hinaus soll kein Nachspülen erforderlich sein.

Die vorgenannte Aufgabe wird erfindungsgemäß gelöst durch im Wesentlichen parfumfreies, alkoholfreies, flüssiges Reinigungs- und Pflegemittel auf Wasserbasis für den menschlichen Intim- und Analbereich sowie für Gegenstände, die damit gegebenenfalls in Kontakt treten, die wenigstens ein Antimikrobium, wenigstens ein hydrophiles Öl, wenigstens einen Schaumstabilisator, wenigstens ein Verdickungsmittel und wenigstens ein Konservierungsmittel enthalten.

Ein erfindungsgemäßes Reinigungs- und Pflegemittel auf Wasserbasis dient zur schonenden hygienischen Reinigung des Intimbereichs sowie des Analbereichs und von Gegenständen, beispielsweise von erotischem Spielzeug. Es reduziert Körpergerüche und wirkt als Infektionsprophylaxe intensiv und mikrobiell gegen Bakterien, Pilze und Viren, beispielsweise Hepatitis B und HIV. Speziell ist das erfindungsgemäße Reinigungs- und Pflegemittel auch für die pflegende Anwendung auf sensibler oder gereizter (Schleim-)Haut geeignet. Eine sichere Anwendung auf allen alkoholempfindlichen Materialien, beispielsweise Latex, Gummi sowie Silikon ist möglich. Das erfindungsgemäße Reinigungs- und Pflegemittel ist dermatologisch getestet, geschmacks- und geruchsneutral.

Das Antimikrobium umfasst beispielsweise Fettsäureamidoalkylbetaine, Antiseptika, wie Chlorhexidin und dessen Derivate sowie kationische, quaternäre Amoniumverbindungen, insbesondere Alkyldimethyl- und Alkydimethylthylbenzylammoniumchloride.

Das hydrophile Öl wirkt als pflegende Komponente und umfasst beispielsweise Polyoxyethylen-Glycerinmonofettsäureester, und Aminoxide.

Als Schaumstabilisatoren werden vorzugsweise gut wasserlösliche, aber nicht hygroskopische Polyethylenglykole eingesetzt, die auch bei Zimmertemperatur flüssig bleiben. Besonders bevorzugt in diesem Sinne ist Dimethylsiloxandigylkolprepolymer, auch unter Dimethiconcopolyol bekannt.

Verdickungsmittel im Sinne der vorliegenden Erfindung umfassen beispielsweise Cocoamidopropyl-Betain auch unter der Bezeichnung Cocoamidopropyldimethylglycin bekannt.

Konservierungsmittel im Sinne der vorliegenden Erfindung werden auch in verdünntem Zustand von der gesunden Haut reaktionslos vertragen. Besonders bevorzugt im Sinne der vorliegenden Erfindung sind Konservierungsmittel sowohl gegen Bakterien als auch gegen Pilze geeignet. Besonders bevorzugt im Sinne der vorliegenden Erfindung ist Ethylenglykolphenylether.

Die Menge an einzusetzendem Antimikrobium richtet sich nach den Gegebenheiten des Einsatzzweckes, sodass im Sinne der vorliegenden Erfindung eine Menge von 0,1 bis 2,0 Gew.-%, insbesondere bis 1,0 Gew.-% des Antimikrobiums in den erfindungsgemäßen Reinigungs-und Pflegemitteln enthalten ist.

Ebenso richtet sich auch die Menge an einzusetzendem hydrophilen Öl nach den Gegebenheiten des Einsatzzweckes des Reinigungs- und Pflegemittels, sodass es im Sinne der vorliegenden Erfindung besonders bevorzugt ist, wenn diese 0,1 bis 5 Gew.-%, insbesondere bis 1,0 Gew.-% hydrophiles Öl enthalten.

Die Menge an Schaumstabilisator im Sinne der vorliegenden Erfindung ist weniger kritisch. Besonders bevorzugt im Sinne der vorliegenden Erfindung ist ein Reinigungs- und Pflegemittel, das 0,1 bis 5 Gew.-%, insbesondere bis 1,0 Gew.-% an Schaumstabilisator enthält.

Die Menge an Verdickungsmitteln in den erfindungsgemäßen Reinigungs- und Pflegemitteln ist dem Anwendungszweck angepasst. Dementsprechend ist es im Sinne der vorliegenden Erfindung besonders bevorzugt, wenn die Reinigungs- und Pflegemittel im Sinne der vorliegenden Erfindung 1 bis 5 Gew.-%, insbesondere bis 1,0 Gew.-% der Verdickungsmittel enthalten.

Die Menge an Konservierungsmitteln im Sinne der vorliegenden Erfindung ist ebenfalls dem Anwendungszweck angepasst und umfasst daher insbesondere 0,1 bis 5 Gew.-%, insbesondere bis 1,0 Gew.-%.

Der zu 100 Gew.-% fehlende Anteil ist im Wesentlichen Wasser. Gegebenenfalls können auch noch Süßungsmittel oder pH-Wert-Regulatoren beispielsweise Citronensäure enthalten sein.

Ein weitere Gegenstand der vorliegenden Erfindung umfasst ein Reinigungs- und Pflegetuch für den menschlichen Intim- und Analbereich sowie für Gegenstände, die damit gegebenenfalls in Kontakt treten, zur Reinigung, Pflege und Desinfektion der Oberflächen aus einer textilen Schicht eines Vlieses, Gewebes oder Gewirkes enthaltend die oben genannten Reinigungs- und Pflegemittel.

Die Reinigungs- und Pflegetücher der vorliegenden Erfindung bestehen vorzugsweise aus Zellstoff, Wolle oder Baumwolle in Form von Vliesen Geweben oder Gewirken. Es können aber auch Kunststoffe wie Polyethylene, Polyester, Polyamide, Polyacrylate und dergleichen verwendet werden.

Ein weiterer Gegenstand der vorliegenden Erfindung umfasst eine Pumpsprühflasche enthaltend die oben genannten Reinigungs- und Pflegemittel.

## Patentansprüche

1. Im Wesentlichen parfumfreies, alkoholfreies, flüssiges Reinigungs-und Pflegemittel auf Wasserbasis für den menschlichen Intim- und Analbereich sowie für Gegenstände, die damit gegebenenfalls in Kontakt treten, enthaltend:
wenigstens ein Antimikrobium,
wenigstens ein hydrophiles Öl,
wenigstens einen Schaumstabilisator,
wenigstens ein Verdickungsmittel und
wenigstens ein Konservierungsmittel.

2. Reinigungs- und Pflegemittel nach Anspruch 1 enthaltend 0,1 bis 1,0. Gew. % Antimikrobium.

3. Reinigungs- und Pflegemittel nach Anspruch 1 enthaltend 0,1 bis 5 Gew. % hydrophiles Öl.

4. Reinigungs- und Pflegemittel nach Anspruch 1 enthaltend 0,1 bis 5 Gew. % Schaumstabilisator.

5. Reinigungs- und Pflegemittel nach Anspruch 1 enthaltend 1,0 bis 5 Gew. % Verdickungsmittel.

6. Reinigungs- und Pflegemittel nach Anspruch 1 enthaltend 0,1 bis 5 Gew. % Konservierungsmittel.

7. Reinigungs- und Pflegetuch für den menschlichen Intim- und Analbereich sowie für Gegenstände, die damit gegebenenfalls in Kontakt treten zur Reinigung, Pflege und Desinfektion der Oberflächen aus einer textilen Schicht eines Vlieses, Gewebes oder Gewirkes enthaltend ein Reinigungs- und Pflegemittel nach einem der Ansprüche 1 bis 6.

8. Pumpsprühflasche enthaltend ein Reinigungs- und Pflegemittel nach einem der Ansprüche 1 bis 6.
